# EUROPEAN PATENT APPLICATION

(11) **EP 3 653 211 A1**
(43) Date of publication of application: **20.05.2020**
(21) Application number: 19213168.8
(22) Date of filing: 28.11.2013
(51) Int. Cl.: A61K 31/495, A61P 25/18

(54) **TRANS-4-{2-[4-(2,3-DICHLOROPHENYL)-PIPERAZIN-1-YL]-ETHYL}-N,N-DIMETHYLCARBAMOYL-CYCLO-HEXYLAMINE FOR TREATING NEGATIVE SYMPTOMS OF SCHIZOPHRENIA**

(30) Priority: 29.11.2012 HU 1200691
(62) Divisional of application: 13805597.5
(71) Applicant: Richter Gedeon Nyrt., 1103 Budapest (HU)
(72) Inventor: Pitter, János György, 2030 Érd (HU); Szatmári, Balázs, 1173 Budapest (HU); Debelle, Marc, 68100 Mulhouse (FR); Németh, György József, 1021 Budapest (HU); Laszlovszky, István, 1111 Budapest (HU)
(74) Representative: Hoffmann Eitle

(57) **Abstract**

The present invention relates to trans-4-{2-[4-(2,3-dichlorophenyl)-piperazin-1-yl]-ethyl}-N,N-dimethylcarbamoyl-cyclohexylamine (cariprazine) and pharmaceutically acceptable salts and hydrates and solvates and polymorphs thereof for use in the treatment of predominantly negative symptoms of schizophrenia.

## Description

### FIELD OF THE INVENTION

The present invention relates to trans-4- {2-[4-(2,3-dichlorophenyl)-piperazin-1 -yl]-ethyl} -N,N-dimethylcarbamoyl-cyclohexylamine (cariprazine) and pharmaceutically acceptable salts and hydrates and solvates and polymorphs thereof for use in the treatment of primary negative symptoms of schizophrenia and/or predominantly negative symptoms of schizophrenia.

### BACKGROUND OF THE INVENTION

Schizophrenia is a prevalent, lifelong disabling psychiatric disorder. The cardinal symptoms of schizophrenia fall into three domains such as positive symptoms (e.g., hallucination, delusion), negative symptoms (e.g., apathy, social withdrawal) and cognitive dysfunction.

The negative symptoms of schizophrenia reflect the absence or diminution of normal behaviors and functions, including problems with motivation, social withdrawal, diminished affective responsiveness, speech, and movement, contribute more to poor functional outcomes and quality of life for individuals with schizophrenia than do positive symptoms.

Distinction can be made between primary and secondary negative symptoms. Primary negative symptoms refer to the symptoms that are intrinsic to schizophrenia, while secondary negative symptoms can be consequent upon several factors including medication side effects (such as extrapyramidal side effects) or depression. Secondary negative symptoms may also be the consequence of positive symptoms: social withdrawal can be caused by persecutory delusions, being distracted and preoccupied by psychotic process, or by a patient titrating down their level of social stimulation to try to minimize intrusive psychotic experiences. (Evidence-based guidelines for the pharmacological treatment of schizophrenia: recommendations from the British Association for Psychopharmacology, Journal of Psychopharmacology 0(0) 1-54)

Secondary negative symptoms would be expected to respond to treatment of the underlying cause. For example, if negative symptoms are secondary to antipsychotic treatment, the symptoms can be decreased by switching to a different antipsychotic with less extrapyramidal adverse effects or by reducing the dosage of the current antipsychotic to a level that does not produce extrapyramidal adverse effects. Similarly, if negative symptoms are secondary to depressed affect, treatments for depression could be considered. If negative symptoms, such as social withdrawal, are caused by immersion in positive symptoms, increasing the dosage of antipsychotic medication or switching to a different antipsychotic may be warranted. If options for treating secondary causes of negative symptoms have failed, the options for pharmacological treatment are limited at present.

Currently atypical antipsychotics are recommended for the treatment of negative symptoms. According to the NICE Guideline (Core Interventions in the Treatment and Management of Schizophrenia in Adults in Primary and. Secondary Care, The National Institute for Health & Clinical Excellence, 2010), negative symptoms can have a major impact on the psychosocial and community functioning of the schizophrenic patients.

Cariprazine is specifically and generically disclosed in WO2005/012266. WO2008/142462 discloses cariprazine for use in the treatment of schizophrenia including negative symptoms of schizophrenia. WO2008/142462 is silent about the origin of negative symptoms described. As it is mentioned above, concerning the effectiveness of a treatment, it is important to distinguish the two types of the negative symptoms. Secondary negative symptoms could be treated by treating the cause but primary negative symptoms remain.

### SUMMARY OF THE INVENTION

The present invention relates to trans-4- {2-[4-(2,3-dichlorophenyl)-piperazin-1-yl]-ethyl}-N,N-dimethylcarbamoyl-cyclohexylamine and hydrates and solvates and polymorphs and pharmaceutically acceptable salts thereof, preferably trans-4- {2-[4-(2,3-dichlorophenyl)-piperazin-1-yl]-ethyl}-N,N-dimethylcarbamoyl-cyclohexylamine hydrochloride for use in the treatment of primary negative symptoms of schizophrenia and/or predominantly negative symptoms of schizophrenia.

### DETAILED DESCRIPTION OF THE INVENTION

We have surprisingly found that trans-4-{2-[4-(2,3-dichlorophenyl)-piperazin-1-yl]-ethyl}-N,N-dimethylcarbamoyl-cyclohexylamine (cariprazine) is very effective in treating primary negative symptoms of schizophrenia.

Primary negative symptoms cannot be assessed as such. To evaluate the effect of a medicinal product on primary negative symptoms it is essential to exclude secondary negative symptoms as much as possible.

As it is mentioned above secondary negative symptoms are mainly the consequence of positive symptoms, extrapyramidal side effects and depression. The less these causes are present the greater the likelihood that primary negative symptoms can be assessed.

For example when negative symptoms are dominant and positive symptoms are presented less prominently (in case of patients with predominantly negative symptoms), it can be presumed that negative symptoms secondary to positive symptoms are less determinant than primary negative symptoms.

According to the clinical study described in Example we have surprisingly found that primary negative symptoms of schizophrenia can be treated effectively with cariprazine.
In the clinical study beside the ITT (intent to treat) population which included the total number of the patients, a subgroup was identified including patients with predominantly negative symptoms. This subgroup was identical with the patient subpopulation showing severe negative symptoms defined by Lenert et al. (Schizophrenia Research 71 (2004) 155-165). Patients showing severe negative symptoms were separated into two groups: State 4 and State 6 (definitions are desribed in Example). State 4 and State 6 together represent the patient subpopulation with predominantly negative symptoms.
As it is mentioned above if negative symptoms are secondary to positive symptoms negative symptoms tend to improve along with the positive symptoms. In case of patients with primary negative symptoms the effect of alleviating negative symptoms can be considered as a direct effect and not as a secondary effect due to the improvement in positive symptoms.If the improvement in negative symptoms is mainly secondary to the improvement in positive symptoms, the improvement on the PANSS factor score for negative symptoms will be expected to decrease or not change in the subpopulation representing patients with predominantly negative symptoms compared to the ITT population.

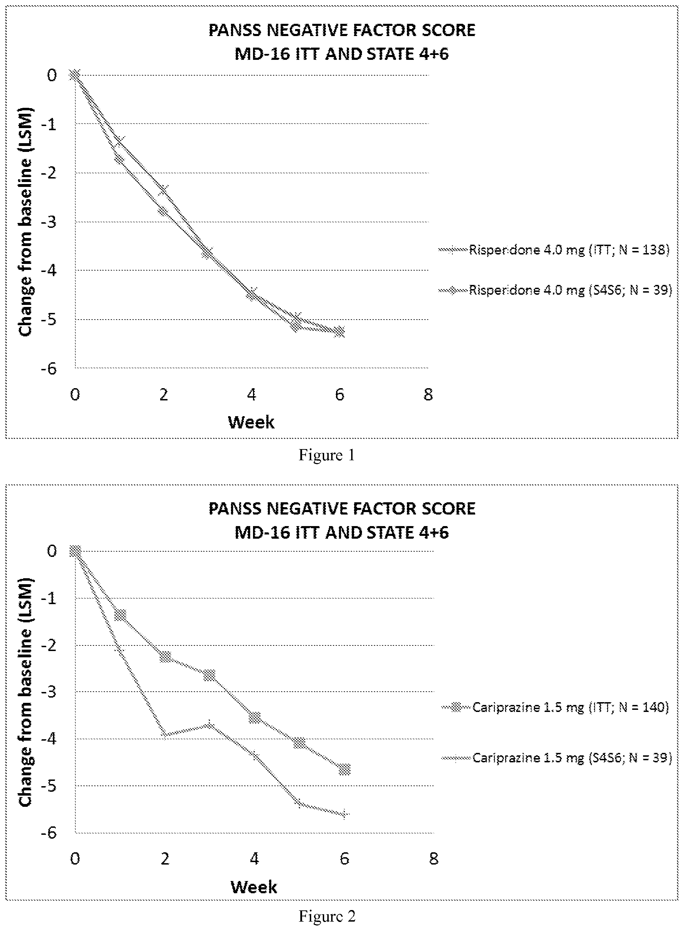

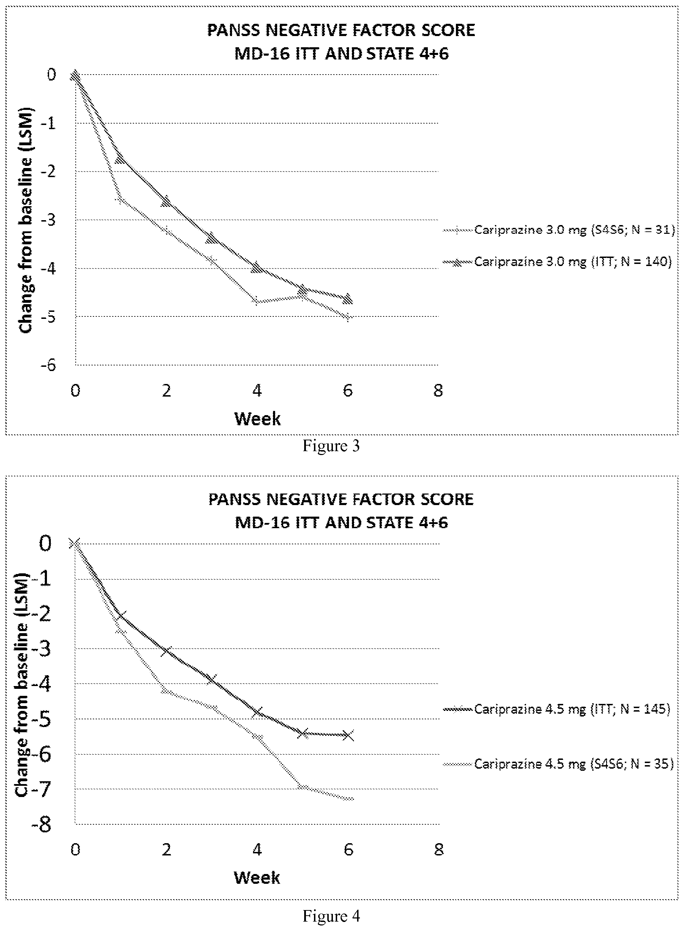

Comparing the improvements on the PANSS factor score for negative symptoms in the ITT population and the subpopulation representing patients with predominantly negative symptoms (Figure 1-4), we have surprisingly found that while there was no difference in risperidone's efficacy on negative symptoms comparing the ITT population to the patient subpopulation showing predominant negative symptoms, cariprazine achieved numerically higher improvement on the PANSS factor score for negative symptoms in the subpopulation with predominant negative symptoms compared to its' effect in the ITT population.

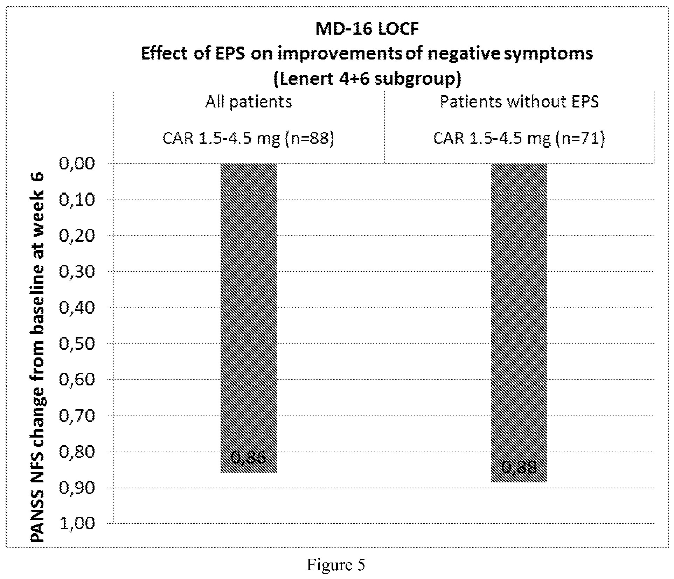

As it was mentioned above extrapyramidal symptoms (EPS) are common side effects of antipsychotic medications. EPS may also cause secondary negative symptoms; hence they could bias the observed effect of cariprazine in negative symptoms. According to their experienced extrapyramidal side effects a subgroup of patients without EPS was selected from the group of patients with predominantly negative symptoms treated with cariprazine. Comparing the efficacy of cariprazine in these groups, it does not seem to be influenced by the treatment emergent EPS (Figure 5).

As it was mentioned above depression can also lead to secondary negative symptoms. In the study, described in Example, the subgroup of patients with predominantly negative symptoms in all cariprazine treatment arms tended to have low to moderate depressive symptoms. It means that they had a maximum score of 4 for question PANSS G6, which rates depression on a scale of 1-7.

The results of this study clearly shows that cariprazine has a direct effect on negative symptoms of schizophrenia. So cariprazine has an effect on primary negative symptoms of schizophrenia.

It has been now discovered that trans-4-{2-[4-(2,3-dichlorophenyl)-piperazin-1-yl]-ethyl}-N,N-dimethylcarbamoyl-cyclohexylamine and pharmaceutically acceptable salts and hydrates and solvates and polymorphs thereof are useful in the treatment of primary negative symptoms of schizophrenia and/or predominantly negative symptoms of schizophrenia.

Pharmaceutically acceptable salts include those obtained by reacting the main compound, functioning as a base with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts can be prepared by reacting the compounds of the invention with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

In a preferred embodiment, the pharmaceutically acceptable salt is a hydrochloride salt.

Some of the compounds useful in the present invention can exist in different polymorphic forms. As known in the art, polymorphism is an ability of a compound to crystallize as more than one distinct crystalline or "polymorphic" species. A polymorph is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds. The use of such polymorphs is within the scope of the present invention.

Some of the compounds useful in the present invention can exist in different solvate forms. Solvates of the compounds of the invention may also form when solvent molecules are incorporated into the crystalline lattice structure of the compound molecule during the crystallization process. For example, suitable solvates include hydrates, e.g., monohydrates, dihydrates, sesquihydrates, and hemihydrates. The use of such solvates is within the scope of the present invention.

Furthermore, the present invention particularly relates to the use of trans-4- {2-[4-(2,3-dichlorophenyl)-piperazin-1 -yl]-ethyl} -N,N-dimethylcarbamoyl-cyclohexylamine and pharmaceutically acceptable salts thereof, more particularly to the use of trans-4-{2-[4-(2,3-dichlorophenyl)-piperazin-1 -yl]-ethyl} -N,N-dimethylcarbamoyl-cyclohexylamine hydrochloride for use in the treatment of primary negative symptoms of schizophrenia.

### DEFINITIONS

The term "predominantly negative symptoms" means that severe negative symptoms are dominant and PANSS factor score for negative symptoms is ≥ 24.

The term "change from baseline" refers to the change of the value of the PANSS factor score for negative symptoms compared to the value registered before treatment started (at the baseline visit). The values were calculated using least squares method. The least squares mean (LSM) values resulted from the analysis of covariance (ANCOVA) model with treatment group and study center as factors and the baseline PANSS factor score of negative symptoms as the covariate.

### Positive and Negative Syndrome Scale (PANSS)

The Positive and Negative Syndrome Scale (PANSS) is a medical scale used for measuring symptom reduction of schizophrenia patients. The scale has seven positive-symptom items (positive subscale), seven negative-symptom items (negative subscale) and, 16 general psychopathology symptom items (general psychopathology subscale). Each item is rated on a scale from 1 (symptom not present) to 7 (symptoms extremely severe). Objectivity and standardization of the scale is optimized by a tightly structured interview. (Kay et al, Schizophr. Bull., 13, 261-76, 1987)

### PANSS factor scores

PANSS factor scores are used in our clinical studies to asses negative, positive and cognitive symptoms of schizophrenia. Each of them is a sum of scores for certain items of the PANSS scale. (Lenert et al.: Schizophrenia Research 71 (2004) 155-165)

PANSS factor score for negative symptoms: Sum of scores for items 1, 2, 3, 4, and 6 in negative subscale: blunted affect, emotional withdrawal, poor rapport, passive social withdrawal, lack of spontaneity; and items 7 and 16 in general psychopathology subscale: motor retardation, and active social avoidance. Higher scores indicate worsening.

PANSS factor score for positive symptoms: Sum of scores for items 1, 3, 5, 6 in positive subscale: delusion, hallucinatory behavior, grandiosity, suspiciousness; and item 9 in general psychopathology subscale: unusual thought content. Higher scores indicate worsening.

PANSS factor score for cognitive symptoms: Sum of scores for items 5, 10, 11, 12, 13 and 15 in general psychopathology subscale: mannrisms and posturing, disorientation, poor attention, lack of judgement and insight, disturbance of volition, preoccupation; and item 2 in positive subscale: conceptual disorganization; and items 5, 7 in negative subscale: difficulty in abstract thinking, stereotyped thinking. Higher scores indicate worsening.

### DESCRIPTION OF FIGURES

**Figure 1****:** Improvements on the PANSS factor score for negative symptoms in the ITT population and the subpopulation representing patients with predominantly negative symptoms in Risperidone (4 mg/day) treatment arm.
**Figure 2****:** Improvements on the PANSS factor score for negative symptoms in the ITT population and the subpopulation representing patients with predominantly negative symptoms in Cariprazine (1.5 mg/day) treatment arm.
**Figure 3****:** Improvements on the PANSS factor score for negative symptoms in the ITT population and the subpopulation representing patients with predominantly negative symptoms in Cariprazine (3 mg/day) treatment arm.
**Figure 4****:** Improvements on the PANSS factor score for negative symptoms in the ITT population and the subpopulation representing patients with predominantly negative symptoms in Cariprazine (4.5 mg/day) treatment arm.
**Figure 5****:** Effect of extrapyramidal symptoms on the change of PANSS factor score for negative symptoms at week 6. Two groups are compared: patients of the Caripazine (1.5 mg/day, 3 mg/day, 4.5 mg/day) treatment arms, patients without EPS of the Caripazine (1.5 mg/day, 3 mg/day, 4.5 mg/day) treatment arms.

The following example is merely illustrative of the present invention and should not be construed as limiting the scope of the invention in any way as many variations and equivalents that are encompassed by the present invention will become apparent to those skilled in the art upon reading the present disclosure.

### EXAMPLE

A representative clinical study was conducted as an international, multicenter, double-bind, placebo- and risperidone-controlled, fixed-dose trial. The objective of the study was to evaluate the safety and efficacy of cariprazine fixed doses in patients with schizophrenia.
A total of 732 patients were selected using criteria that includes patients who (i) currently meet or have met in the past the *Diagnostic and Statistical Manual of Mental Disorders, Fourth Edition, Text Revision (DSM-IV-TR)* criteria for schizophrenia (295.30 Paranoid Type, 295.10 Disorganized Type, 295.20 Catatonic Type, or 295.90 Undifferentiated Type) based on the Structured Clinical Interview for DSM-IV (SCID), (ii) have a PANSS total score ≥ 80 and ≤ 120, (iii) have a score ≥ 4 on Clinical Global Impression-Severity scale (iv) have a score ≥ 4 on at least 2 of the following 4 PANSS positive symptoms: delusions, hallucinatory behavior, conceptual disorganization, and suspiciousness/persecution.
During a 6 week period, 3 doses of cariprazine (1.5 mg/day, 3 mg/day, 4.5 mg/day) was compared to placebo and to an effective dose of risperidone (4.0 mg/day).
Beside the ITT (intent to treat) population which included the total number of the patients, a subgroup was identified including patients with predominantly negative symptoms. This subgroup was identical with the patient subpopulation showing severe negative symptoms defined by Lenert et al. (2004). Lenert et al. divided schizophrenia into 8 states, each based on a three-axis scale (PANSS positive, negative and cognitive factor scores). Patients showing severe negative symptoms were separated into two groups: State 4 (severe with negative dominance of symptoms) and State 6 (severe with negative and cognitive symptoms). The definition of State 4 is the following:
- PANSS factor score for negative symptoms is ≥ 24
- PANSS factor score for positive symptoms is ≤ 19
- PANSS factor score for cognitive symptoms is ≤ 26
The definition of State 6 is the following:
- PANSS factor score for negative symptoms is ≥ 24
- PANSS factor score for positive symptoms is ≤ 19
- PANSS factor score for cognitive symptoms is ≥ 27

## Claims

1. Trans-4-{2-[4-(2,3-dichlorophenyl)-piperazin-1-yl]-ethyl}-N,N-dimethylcarbamoyl-cyclohexylamine and/or pharmaceutically acceptable salts and/or hydrates and/or solvates and/or polymorphs thereof for use in treating predominantly negative symptoms of schizophrenia.

2. Trans-4-{2-[4-(2,3-dichlorophenyl)-piperazin-l-yl]-ethyl}-N,N-dimethylcarbamoyl-cyclohexylamine for use according to claim 1, in the form of trans-4-{2-[4-(2,3 - dichlorophenyl)-piperazin-l-yl]-ethyl}-N,N-dimethylcarbamoyl-cyclohexylamine hydrochloride and/or hydrates and/or solvates and/or polymorphs thereof.
